# EUROPÄISCHE PATENTSCHRIFT

(11) **EP 2 641 095 B1**
(45) Veröffentlichungstag und Bekanntmachung des Hinweises auf die Patenterteilung: **10.01.2018**
(21) Anmeldenummer: 11793695.5
(22) Anmeldetag: 17.11.2011
(51) Int. Cl.: C12M 1/12, B01L 1/02, B01L 7/00, C12M 1/22, C12M 3/00, C12M 1/00, G01N 35/00, G01N 35/02, G01N 35/04

(54) **KLIMATISIERUNGSRAUM FÜR EINE ZEITGESTEUERTE LAGERUNG VON PROBEN UND VERFAHREN ZUR ZEITGESTEUERTEN LAGERUNG VON PROBEN**
AIR-CONDITIONING SPACE FOR STORING SAMPLES IN A TIME-CONTROLLED MANNER AND METHOD FOR STORING SAMPLES IN A TIME-CONTROLLED MANNER
ENCEINTE DE CONDITIONNEMENT POUR LE STOCKAGE D'ÉCHANTILLONS AVEC CONTRÔLE DU TEMPS ET PROCÉDÉ DE STOCKAGE D'ÉCHANTILLONS AVEC CONTRÔLE DU TEMPS

(30) Priorität: 17.11.2010 DE 102010060634
(43) Veröffentlichungstag der Anmeldung: 25.09.2013
(73) Patentinhaber: Andreas Hettich GmbH & Co. KG, 78532 Tuttlingen (DE)
(72) Erfinder: EBERLE, Klaus-Günter, 78532 Tuttlingen (DE); CLARKE, Gavin, 78532 Tuttlingen (DE)
(74) Vertreter: Puschmann Borchert Bardehle Patentanwälte Partnerschaft mbB
(86) Internationale Anmeldenummer: PCT/EP2011/070399
(87) Internationale Veröffentlichungsnummer: WO 2012/066101

(56) Entgegenhaltungen:
- EP-A1- 0 293 782
- EP-A2- 1 757 882
- EP-A2- 1 757 882
- WO-A1-91/19199
- WO-A1-91/19199
- WO-A1-2010/001873
- WO-A2-02/059251
- DE-A1- 10 009 555
- DE-B4- 10 304 012
- US-A1- 2003 031 602
- US-A1- 2004 256 963
- US-A1- 2004 256 963
- US-B1- 6 475 776
- US-B1- 6 475 776

## Beschreibung

Die Erfindung betrifft einen Klimatisierungsraum für eine zeitgesteuerte Lagerung von Proben gemäß der im Oberbegriff des Anspruchs 1 angegebenen Art.

Nach dem Stand der Technik sind automatische Zuführeinrichtungen für Inkubatoren bekannt, bei welchen packenweise Proben in Probenschalen in einen Inkubationsraum eingeführt werden, um eine möglichst hohe Lagerdichte zu erreichen. Für eine zeitgesteuerte Lagerung sind Umladesysteme im Inneren des Inkubationsraums integriert, beispielsweise Paternoster- oder Förderbandsysteme.

Diese Lösungen haben den Nachteil, dass zwar eine hohe Packdichte erreichbar ist, jedoch zum einen, die in den Inkubator integrierten Fördersysteme bei Kontamination schwer zu reinigen sind, zum anderen keine Individuelle Entnahme einzelner Proben ermöglicht wird. Die Entnahme ist nur gemäß dem Förderfortschritt des Fördersystems sowie nur in der durch das Fördersystem vorgegebenen Reihenfolge möglich.

Die EP 0 293 782 A1 offenbart einen Inkubator, der ein als Karussell ausgebildetes Lagersystem umfasst.

Die DE 103 04 012 B4, in welcher ein Klimaschrank mit einer Objektlagervorrichtung und einer inneren Transportvorrichtung offenbart ist. Gemäß dieser Druckschrift ist vorgesehen, dass ein einzulagerndes Objekt von einer äußeren Transportvorrichtung an eine innere Transportvorrichtung übergeben wird und eine innere Transportvorrichtung das Objekt an seiner vorbestimmten Ablageposition einlagert.

Die WO 02/059 251 A2 offenbart ebenfalls einen klimatisierten Lagerschrank, der ein in vertikaler Richtung festes, aber drehbares Rondell / Karussell aufweist, das über eine Zufuhreinrichtung automatisch geladen werden kann. In dieser Ausführungsform ist die Zuführeinrichtung/Beladeeinrichtung innerhalb des klimatisierten Schranks angeordnet.

Die DE 100 09 555 A1 offenbart ebenfalls einen automatisch beladbaren Klimaschrank. Dieser weist ebenfalls in vertikaler Richtung ein starres Lagersystem auf. Ferner umfasst der Klimaschrank eine innerhalb des Klimaschranks angeordnete Transporteinrichtung. Diese Transporteinrichtung legt die Proben, nach der Einführung in den Klimaschrank, in den dafür vorgesehenen Fächern ab. Die US 6 475 776 offenbart einen Inkubator mit einem Karussell welches der Höhe nach verfahrbar ist und eine Durchführung zum Einbringen von Proben aufweist. Durch die Höhenverstellbarkeit des Probenkarussells befindet sich innerhalb ein großer ungenutzter Raum welcher ebenfalls klimatisiert werden muss.

Die US 2003/0031 602 offenbart ein Probenkarussell innerhalb eines klimatisierten Raumes mit einer vielzahl von Öffnungen zur Durchführung von Proben.

Die genannten Ausführungen haben den Vorteil, dass die Transportvorrichtungen, die im Innenraum des Inkubators angeordnet sind problemlos die verschiedenen Lagerpositionen, die sich insbesondere in ihrer vertikalen Lage unterscheiden anzufahren.
Sie haben jedoch den Nachteil, dass eine Transporteinrichtung innerhalb des Klimaschranks vorgesehen sein muss, welche aufgrund der Antriebe die Bedingungen innerhalb des Klimaschranks negativ beeinflusst. Dies kann einerseits durch Abrieb von mechanischen Komponenten, zum anderen durch die Wärmeentwicklung der Motoren geschehen.
Es ist Aufgabe der Erfindung, eine Vorrichtung anzugeben, die es ermöglicht eine hohe Packdichte, bei einfachen Reinigungseigenschaften und individuell zeitgesteuerter Entnahme zu ermöglichen.
Die Aufgabe wird durch die kennzeichnenden Merkmale des Anspruchs 1 in Verbindung mit den Oberbegriffsmerkmalen gelöst.
In bekannter Weise umfasst ein Klimatisierungsraum für eine zeitgesteuerte Lagerung eine automatische Zuführ- und Entnahmeeinrichtung von Probenbehältern in einen klimatisch abgeschlossenen Raum mit zumindest einer Wand. Die Probenbehälter sind durch zumindest eine Öffnung in den klimatisch abgeschlossenen Raum einführbar. Die automatische Zuführeinrichtung weist zumindest eine Antriebs- und Steuereinheit auf. Ferner ist eine Inkubationsaufnahme für die Probenaufnahme im Inneren des Klimatisierungsraums vorgesehen. Die Inkubationsaufnahme ist in vertikaler Erstreckung fest.
Erfindungsgemäß umfasst Zuführ-/Entnahmeeinrichtung ein Greifmittel, mit dem ein Probenbehälter von einer Aufnahmeposition außerhalb des klimatisch abgeschlossenen Raums, in einer eindeutigen Ablageposition ablegbar ist. Dabei ist die Zuführeinrichtung außerhalb des Klimatisierungsraums angeordnet. Die Probenbehälter sind durch das Greifmittel jeweils einzeln entnehmbar.

Durch die einzelne Zuführung der Probenbehälter und deren Ablage in einer eindeutigen Aufnahmeposition mittels des Greifmittels ist es beispielsweise möglich, dass die in den Klimaschrank eingebrachten Probenbehälter nach individuellen Zeitvorgaben inkubiert werden können und eine Zwischenauswertung des momentanen Probenzustands ermöglicht wird.

Indem die Ablage durch einen von außen in den Klimaraum eingeführten Greifmittels erfolgt, kann die Inkubationsaufnahme sehr einfach gestaltet sein, wodurch sie entsprechendleicht zu reinigen ist. Durch die Anordnung der Zuführeinrichtung außerhalb des klimatisierten Raums wird zudem eine Kontamination des klimatisierten Raums von vornherein unterbunden sowie ein Wärme- oder Kälteeintrag in diesen vermieden.

In einer ersten vorteilhaften Ausgestaltung kann das Greifmittel ein Gelenk aufweisen, welches dafür sorgt, dass der in der Aufnahmeposition gegriffene Probenbehälter, gedreht in der Inkubationsaufnahme ablegbar ist. Die auszuführenden Drehbewegungen des Probenbehälters beanspruchen je nach Geometire des Probenbehälters viel Raum. Indem die Zuführ- / Entnahmeeinrichtung und das Greifmittel außerhalb des Klimaschranks angeordnet sind, muss für derartige Drehungen des Probenbehälters kein klimatisierter Raum zur Verfügung gestellt werden. Eine Ausrichtung des Probenbehälters kann somit außerhalb des Klimaschrnaks durch die Zuführ-/Entnahmeeinrichtung erfolgen.

Auf diese Weise können Proben schnell in der korrekten Orientierung abgelegt werden.
Ein Vorteil ergibt sich durch diese Maßnahme vor allem bei der Verwendung von auf Petrischalen gezüchteten Kulturen, da sich durch die Klimatisierung bildendes Kondenswasser nicht auf den in der Petrischale liegenden Nährboden tropft und das Kulturenwachstum beeinträchtigt, sondern im Deckel verbleibt bzw. aufgefangen wird, bis es sich verflüchtigt. Dies sorgt für eine höhere Zuverlässigkeit und bessere Probenqualität.

Indem die Probe automatisch gedreht wird, ist ein menschlicher Eingriff in die Weiterbehandlung der Proben, nachdem der Nährboden mit der Kultur bestrichen wurde, nicht mehr notwendig. Menschliche Fehler werden ausgeschlossen. Zum einen ermöglicht dies eine optimale Inline-Behandlung in Verbindung mit einer anschließbaren Vorrichtung zum automatischen Probenauftrag, zum anderen beugt dies Fehlern in der Lagerung vor.

Zur weiteren Verbesserung der Sicherheit kann eine Lagebestimmungsvorrichtung vorgesehen sein, welche die Lage eines Probenbehälters, insbesondere einer Petrischale, in der Aufnahmeposition bewertet. Der Probenbehälter wird folglich nur im Bedarfsfall durch den Greifer gewendet in den klimatisch abgeschlossenen Raum eingebracht. Liegt ein Probenbehälter bereits in gewendeter Lage in der Aufnahmeposition kann dieser entweder in den klimatisch abgeschlossenen Raum eingebracht werden ohne abermals gewendet zu werden, oder zur Begutachtung durch Fachpersonal aussortiert werden. Die Lagebestimmungsvorrichtung, kann abhängig von der Ausgestaltung der verwendeten Petrischalen eine einfache Lichtschranke sein.

In einer besonders vorteilhaften Ausführungsform, ist der mit einem Zuführarm verbundene Greifer auf einem Verfahrmittel angeordnet, durch das der Greifer in seiner vertikalen Lage verfahren werden kann. Dies erlaubt die Ablage der aufgenommenen Probe auf unterschiedlichen Ebenen und erhöht dadurch die Anzahl der eindeutig ablegbaren Proben signifikant, ohne eine komplexe Gestaltung der Inkubatoraufnahme zur Folge zu haben. Zudem kann eine höherer Zugriffsgeschwindigkeit erreicht werden, indem der Greifer in seiner vertikalen Position angepasst wird, und nicht die mit Proben beladenen Inkubatoraufnahmen. Ferner kann der Greifer auf einer horizontalen bewegbaren Unterlage oder einem horizontal bewegbaren Zuführarm angebracht sein, um eine Probe horizontal in den klimatisierten Raum einzufahren.

Zur Zeitdatengeneration kann ein Barcodeleser im Aufnahmebereich an der Aufnahmevorrichtung vorgesehen sein, der abhängig von einem auf der Probe befindlichen Barcode eine individuelle Zeiteinstellung zur Lagerung für den einzelnen Probenbehälter ermöglicht. Über diese Barcode-Kennzeichnung oder ähnliche Kennzeichnung kann auch eine eindeutige, der Kennzeichnung zugeordnete vollautomatische Ablage ermöglicht werden. Zudem kann überprüft werden, ob die Probe in den richtigen klimatisierten Raum eingebracht und dort behandelt wird.

Die Schnittstelle, die sich zwischen klimatisiertem Raum und unklimatisiertem Raum ergibt, kann im Wesentlichen derart ausgestaltet sein, dass in der Abtrennung zwischen klimatisiertem Raum und unklimatisiertem Raum grundsätzlich eine Ausnehmung vorgesehen ist, die eine Durchführung des Zuführarms/Greifers in den zu klimatisierenden Raum ermöglicht.

Die Ausnehmung kann sich über die gesamte Höhe des Inkubators erstrecken, wodurch sich eine Beladungsmöglichkeit des Inkubators über diesen Bereich ergibt. Um eine ideale Klimatisierung des klimatisierten Raums zu ermöglichen, aber auch um unerwünschten Wärme- oder Kälteverlust und somit Energieverschwendung zu verhindern, ist eine Abdeckung der vertikalen Ausnehmung vorgesehen, die im Bedarfsfall geöffnet werden kann.

Vorzugsweise ist die Abdeckung so gestaltet, dass diese nur eine absolut notwendige Öffnung unabgedeckt lässt und diese Öffnung entsprechend der Zuführung oder Herausnahme von Probenbehältern vertikal verfahrbar ist. Eine Umsetzung kann so gestaltet sein, dass ein Durchführungskanal an seinem oberen und seinem unteren Ende an eine Rollo-Abdeckung angeschlossen ist, welche über eine untere und eine obere Aufwickelrolle geführt ist. Auf diese Weise wird ein größtmöglicher vertikaler Verfahrbereich mit minimaler Öffnung des Durchführungskanals erzielt. Die Klimakonditionen werden durch die Öffnung nicht negativ beeinflusst.

Als zusätzliche Sicherungsmaßnahme kann ein Sensor vorgesehen sein, der überprüft, ob die von der Datenverarbeitungseinrichtung zugewiesene Ablageposition auch tatsächlich frei ist.

Gemäß einer Weiterbildung der Erfindung wird ein Verschluss des Durchführungskanals vorgeschlagen, der nach der Entnahme oder Zuführung den Durchführungskanal wieder verschließt.

In einer weiteren vorteilhaften Ausführungsform ist die Inkubatoraufnahme als drehbares Karussell ausgebildet, wodurch eine bessere Flächenausnutzung bei vereinfachter Mimik ermöglicht wird. Ein Karussell erlaubt es, dass der Greifer nur eine Position in der Nähe der Ausnehmung anfahren muss, um dennoch eine hohe Packdichte zu erreichen, da durch die Drehung des Karussell immer eine bestimmte Position bestückt oder entnommen werden kann. In einer vorteilhaften Weise wird die Position einer Probe mittels einer Datenverarbeitungseinrichtung indiziert, um so einen eindeutigen schnellen Zugriff zu ermöglichen. So können beispielsweise für die Ablage in einem Karussell folgende Parameter festgelegt werden. Zum einen ein Drehwinkel des Karussell und zum anderen die Ebene, was eine eindeutige Zuordnung einer Probe zu einer Positionermöglicht.

In einer besonders vorteilhaften Ausführungsform werden die Proben durch eine Petrischale gebildet.

Der Durchführungskanal kann vorzugsweise eine Öffnung aufweisen, durch welche die Petrischale in horizontaler Lager durchführbar ist. Analog ist auch die Inkubationsaufnahme für die Aufnahme der Petrischalen in einer horizontalen Position ausgerichtet. Die Petrischalen können nur im Umfangsbereich der einzelnen Karussell Einlagen eingelegt werden. Dies hat den Vorteil, dass die Zuführung sehr einfach ausgestaltet sein kann, und dennoch eine Eindeutigkeit gewährleistet ist, andererseits, eine hohe Packdichte unter den gegebenen Rahmenbedingungen erzielt werden kann. Um eine automatische Analyse und Bewertung der Proben zu gewährleisten, kann im Bereich der Zuführeinrichtung ein Tischdetektor, insbesondere eine CCD-Kamera angeordnet sein, welche die Proben vor der Zuführung überprüft und das gewonnene Ergebnis auf einer Datenverarbeitungsanlage ablegt. Ferner können Proben nach einer vorgegebenen Zeit aus dem klimatisierten Raum entnommen werden und abermals der Analysevorrichtung zugeführt werde um einen Vergleich der Probenentwicklung während der Lagerungszeit zu ermöglichen.
Diese Digitalisierung der Probenanalyse hat den Vorteil, dass sie auf einem Server von einer Vielzahl von Personen, insbesondere online abgefragt werden kann oder auch an bestimmte Personen geschickt werden kann.
Ferner können gemäß einem Verfahren zur zeitgesteuerten automatischen Lagerung von Proben in einem klimatisierten Raum, Probenbehälter automatisch in den klimatisierten Raum eingebracht werden und nach einer bestimmten Zeit wieder aus dem klimatisierten Raum entnommen werden. Es kann jedem Probenbehälter zumindest eine individuelle Zeitspanne zugewiesen werde, nach welcher der Probenbehälter Probe nach Lagerung wieder entnommen wird, wobei jedem Probenbehälter mit Einbringen ein freier Platz in einem Lagersystem zugewiesen wird.
In einem vorteilhaften Verfahrensschritt, können die Probenbehälter vor der Zuführung in den klimatisierten Raum gewendet werden.
Vorzugsweise werden mit einem Barcode versehene Proben vor der Lagerung ausgelesen. Abhängig von den in dem Barcode vorhandenen Informationen werden diese an eine
Datenverarbeitungseinrichtung zur Weiterverarbeitung übermittelt. Der Barcode kann Informationen über die Lagerintervalle, die relevanten Ansprechpartner, oder die Zuordnung zu einem speziellen Klimatisierungsraum enthalten.
Besonders vorteilhaft ist es, dass überprüft werden kann, ob die für den Probenbehälter bestimmte Lagerposition tatsächlich nicht bereits von einem Probenbehälter belegt ist.

Ein weiterer Schritt in der Probenbehandlung kann vorsehen, dass zumindest vor dem ersten Einbringen der Probenbhälter in den klimatisierten Raum diese überprüft werden, und das Ergebnis der Überprüfung an eine Datenverarbeitungsanlage übermittelt wird.

Die Überprüfung kann mit einer Kamera erfolgen, die beispielsweise einen Probenzustand dokumentiert.

Weitere Vorteile, Merkmale und Anwendungsmöglichkeiten der vorliegenden Erfindung ergeben sich aus der nachfolgenden Beschreibung in Verbindung mit den in den Zeichnungen dargestellten Ausführungsbeispielen.

In der Beschreibung, in den Ansprüchen und in der Zeichnung werden die in der unten aufgeführten Liste der Bezugszeichen verwendeten Begriffe und zugeordneten Bezugszeichen verwendet. In der Zeichnung bedeutet:
- Fig. 1: eine perspektivische Ansicht eines erfindungsgemäßen Inkubators,
- Fig. 2: eine perspektivische Ansicht eines erfindungsgemäßen Inkubators, wobei die Seitenwände des Inkubators aus Gründen der Übersicht nicht dargestellt sind, und
- Fig. 3: eine Detailansicht eines erfindungsgemäßen Inkubators.

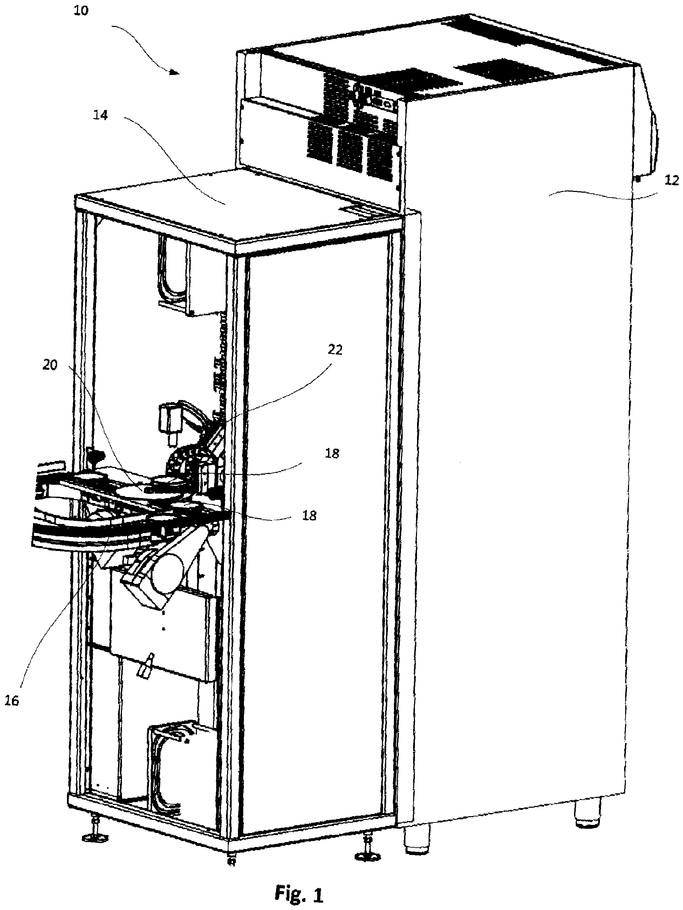

Fig. 1 zeigt einen erfindungsgemäßen Inkubator 10 umfassend eine Klimatisierungschrank 12 sowie einen nicht klimatisierten Schrank 14 sowie eine Anschlussstelle an ein Fördersystem 16.

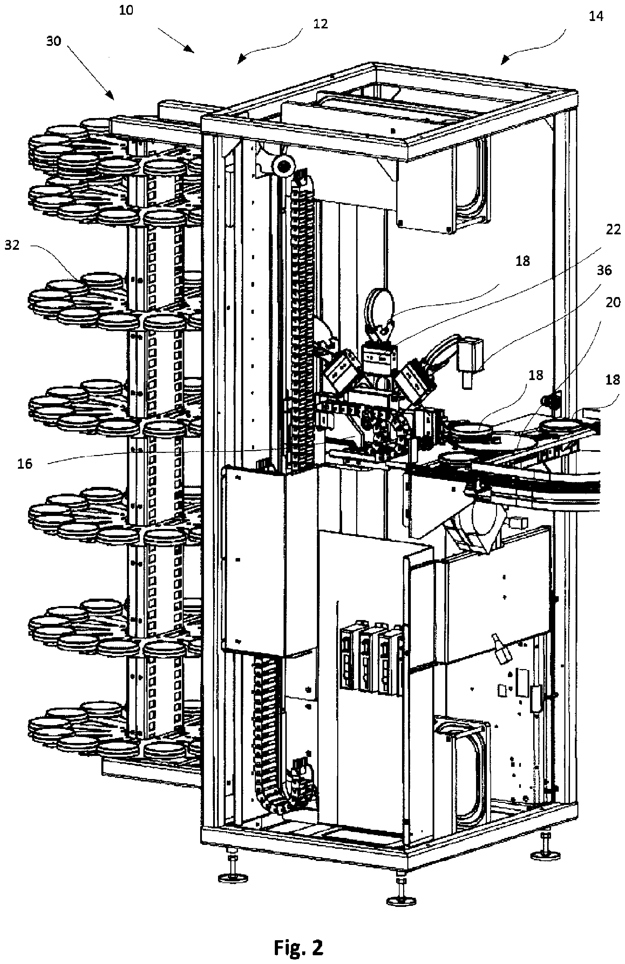

Die über das Fördersystem 16 geförderten Petrischalen 18 werden von einer Separationseinheit 20 durch Drehen in eine Aufnahmeposition 21 gebracht. Dort werden Sie von einem Greifarm 22 erfasst, angehoben, von einer Kamera 48 erfasst und dann in den klimatisierten Raum 12 gebracht und dort abgelegt. Die nähere Funktionsweise wird anhand von Fig. 2 beschrieben.

Fig. 2 zeigt eine Darstellung des Inkubators 10, wobei die Seitenwände nicht dargestellt sind. Die Petrischalen 18 werden vom Greifarm 22 aufgenommen, wobei diese bei der Zuführung in den Inkubationsraum, also den klimatisierten Raum 12, gewendet werden. Im Inkubationsraum 12 ist eine Aufnahmevorrichtung vorgesehen, in welche die Petrischalen 18 eingelegt werden. Diese Aufnahmevorrichtung ist als Karussell ausgebildet und umfasst einen drehbaren Holm 32 wobei an diesem Holm 32 einzelne Tablare 34 lösbar angebracht sind. Diese Tablare 34 können je nach Bedarf in verschiedenen Höhen mit verschiedenen Abständen zueinander am Holm 32 angebracht werden.

Die Petrischalen 18 werden durch einen Durchführungskanal 40 an einen freien Platz gegenüber des Durchführungskanals 40 gebracht.Die Petrischalen 18 werden durch den Zuführarm 22 mit Greifer 24 in den Inkubationsraum transportiert. Die Inkubatoraufnahme 30 ist über einen Motorantrieb drehbar gelagert, wodurch ermöglicht wird, dass ein Tablar 34 derart positioniert werden kann, dass ein freier Platz auf dem Tablar 34 dem Durchführungskanal 40 gegenüberliegt.

Auf diese Weise kann der an einem Zuführarm 22 angeordnete Greifer 24 eine Petrischale 18 über einen sehr kurzen Verfahrweg eindeutig im klimatisierten Raum 12 ablegen.

Ferner ist eine vertikale Verfahreinheit 28 vorgesehen, auf welcher der Zuführarm 22 mit Greifer 24 montiert ist. Dies ermöglicht, dass der Zuführarm 22 mit Greifer 24 die Petrischalen 18 in verschiedenen Ebenen der Inkubatoraufnahme 30 ablegen kann. Ferner ist ein Barcode-Leser 36 vorgesehen, der die gekennzeichneten Petrischalen 18 identifiziert und diese Information an eine hier nicht dargestellte Datenverarbeitungsanlage weitergibt. Die Datenverarbeitungsanlage weist einer Petrischale eine Position in der Inkubatoraufnahme 30 zu. Entsprechend dieser Position wird der Zuführarm 22 mit Greifer 24 in vertikaler Richtung verfahren und die Inkubatoraufnahme 30 ist derart positioniert, dass der zugewiesene Ablageplatz nächstliegend zum Durchführungskanal 40 des Greifers 24 liegt. Neben der Positionierung werden den einzelnen Probenbehältern Zeitwerte zugeordnet, die auch im Barcode codiert sein können, z. B. wie lange die einzelnen Proben im Inkubator gelagert werden sollen, und ob Zwischenanalysen für die einzelnen Proben notwendig sind.

Hat eine Probe ihren Zeitwert erfüllt, fährt der Zuführarm 22 mit Greifer 24 und die Inkubatoraufnahme 30 wieder eine entsprechende Position an, in der die Petrischale 18 aus dem klimatisierten Raum 12 entnommen werden kann. Der Probenbehälter 18 wird mittels des Zuführarms 22 mit Greifer 24 wieder gedreht auf dem Separationselement 20 abgelegt, worauf dieses in der Prozessstraße weitergeführt werden kann. Vor dem Ablegen überprüft der Barcode-Leser 36, ob die richtige Petrischale 18 entnommen wurde. Dies sorgt für eine zusätzliche Fehlervermeidung.

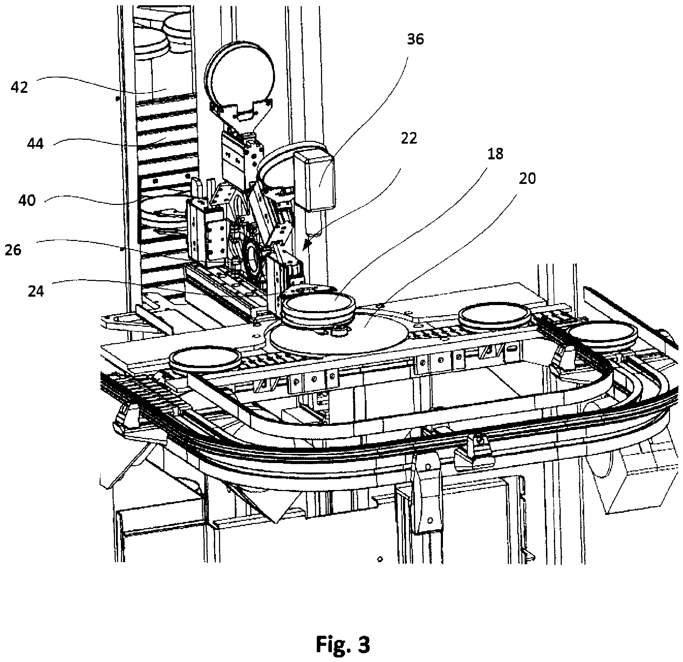

Fig. 3 zeigt eine Detailaufnahme des Zuführarms 22 mit Greifer 24 in einem Bewegungsbild, wobei deutlich wird, dass der Zuführarm 22 mit Greifer 24 auf einem Lager 26 angeordnet ist. Durch eine Verschwenken des Greifers 24 wird eine Drehung der Petrischale 18 um 180° ermöglicht. Dieses Wenden der Petrischale 18 hat den Vorteil, dass sofern sich Kondenswasser während der Klimatisierung in den Petrischalen 18 bildet, dieses nicht auf dem Nährboden der Probe verbleibt, sondern auf den Deckel abtropfen kann.

Ferner ist in dieser Darstellung der Durchgangskanal 40 dargestellt, der die Zuführung in den Inkubationsraum 12 ermöglicht. Dieser ist in seinen Abmessungen so gewählt, dass gerade die Petrischale 18 durch den Durchführungskanal 40 geschoben werden kann. Um die Durchführung in verschiedenen vertikalen Positionen zu ermöglichen, ist in der Seitenwand 43 im Übergang von klimatisierten zum nicht klimatisierten Raum eine vertikale Ausnehmung 42 eingebracht, die jedoch von einem hier angedeuteten Rollosystem 44 überdeckt wird. Dieses Rollosystem 44 ist am Durchgangskanal 40 sowohl unten als auch oben befestigt. Am unteren und oberen Ende der vertikalen Ausnehmung sind Spannrollen vorgesehen, die bei einem nach oben bzw. nach unten Fahren des Durchgangskanals 40 das Rollosystem 44 aufrollen bzw. abrollen. Der Durchgangskanal 40 ist mit der vertikalen Verfahreinheit 28, auf welcher der Zuführarm 22 mit Greifer 24 angeordnet ist, verbunden. Der Durchgangskanal 40 liegt dadurch immer in der richtigen Position für den Zuführarm 22 mit Greifer 24. Befindet sich der Greifer 24 in der korrekten vertikalen Position muss dieser lediglich durch den Durchführungskanal 40 in den Inkubationsraum 12 mit dem Zuführarm 22 über die vertikale Verfahreinheit 28 eingefahren werden und in die nächstliegende Aufnahme der Inkubatoraufnahme 30 abgelegt werden.

Durch die erfindungsgemäße Ausbildung eines Klimatisierungsraums kann ein automatisiertes individualisiertes Probenhandling erreicht werden, wodurch die Möglichkeit eröffnet wird, nach individuellen Zeitangaben die Probenbehälter 18 vollautomatisch in den Inkubator einzubringen und wieder zu entnehmen. Abgesehen davon besteht durch die individualisierte Zuführung und Entnahme die Möglichkeit automatisch Zwischenkontrollen der Proben durchzuführen.

### Bezugszeichenliste

- 10: Klimaraum
- 12: Klimatisierungsschrank
- 14: Unklimatisierter Schrank
- 16: Fördersystem
- 18: Petrischale, Probenbehälter
- 20: Separationseinheit
- 21: Aufnahmeposition
- 22: Zuführarm24 Greifer
- 26: Lager
- 28: vertikal verfahrbare Lagerung
- 30: Inkubatoraufnahme
- 32: Holm
- 34: Tablar
- 36: Barcode-Leser
- 40: Durchgangskanal
- 42: Ausnehmung
- 43: Seitenwand
- 44: Rollosystem
- 48: Kamera, Detektionseinheit

## Patentansprüche

1. Klimatisierungsraum (10) für eine zeitgesteuerte Lagerung von Proben umfassend eine automatische Zuführeinrichtung (22) von Probenbehältern (18) in einen klimatisch abgeschlossenen Raum (12) mit zumindest einer Wand (43), in den ein Probenbehälter (18) durch eine Öffnung (40,42) in den klimatisch abgeschlossenen Raum (12) einführbar ist, wobei die Zuführeinrichtung (22) zumindest eine Antriebs- und Steuereinheit aufweist, und eine Inkubatoraufnahme (30) für die Aufnahme der Proben (18) im Inneren des klimatisch abgeschlossenen Raums (12) vorgesehen ist, wobei die Zuführeinrichtung einen automatischen Zuführarm (22, 24) aufweist, der einen Probenbehälter (18) von einer Aufnahmeposition außerhalb des klimatisch abgeschlossenen Raums (12) greift, und den Probenbehälter (18) dort in einer eindeutigen Ablageposition in einer Inkubatoraufnahme (30) ablegt, wobei der Zuführarm (22, 24) außerhalb des Klimatisierungsraums (12) angeordnet ist, und die Probenbehälter (18) einzeln aus dem klimatisch abgeschlossenen Raum (12) mit dem Zuführarm (22, 24) entnehmbar sind, **dadurch gekennzeichnet, dass** die Öffnung durch einen Durchführungskanal (40) gebildet ist, der zumindest vertikal verschiebbar ist.

2. Klimatisierungsraum nach Anspruch 1, **dadurch gekennzeichnet, dass** der Zuführarm (22, 24) derart gelagert ist, dass ein Probenbehälter (18) während des Transports von der Aufnahmeposition zur Ablageposition gewendet wird.

3. Klimatisierungsraum nach einem der vorangehenden Ansprüche, **dadurch gekennzeichnet, dass** der Zuführarm (22, 24) auf einem Verfahrmittel (28) angeordnet ist, wodurch dieser in vertikaler Richtung verfahrbar ist und somit auf eine Ablageposition auf verschiedenen Ebenen der Inkubatoraufnahme (30) zugreifen kann.

4. Klimatisierungsraum nach einem der vorangehenden Ansprüche, **dadurch gekennzeichnet, dass** eine Aussparung (42) in vertikaler Erstreckung in der Seitenwand (43) vorgesehen ist.

5. Klimatisierungsraum nach einem der vorangehenden Ansprüche, **dadurch gekennzeichnet, dass** die Inkubatoraufnahme als drehbares Karussell (30) ausgebildet ist.

6. Klimatisierungsraum nach Anspruch 5, **dadurch gekennzeichnet, dass** ein Motor außerhalb des Raums (12) vorgesehen ist, der durch Übertragungsmittel das drehbare Karussell (30) antreibt.

7. Klimatisierungsraum nach einem der vorangehenden Ansprüche, **dadurch gekennzeichnet, dass** der klimatisch abgeschlossene Raum (12) ein Brutschrank, Klimaschrank oder Inkubator, insbesondere zum Kühlen und/oder Heizen, ist.

8. Klimatisierungsraum nach einem der vorangehenden Ansprüche, **dadurch gekennzeichnet, dass** die Aufnahmeposition in einer Anschlussstelle (20) an eine Förderlinie (16) liegt.

9. Klimatisierungsvorrichtung nach einem der vorangehenden Ansprüche, **dadurch gekennzeichnet, dass** ein Barcodeleser (36) vorgesehen ist, der im Barcode hinterlegte Informationen auf dem Probenbehälter (18) auswerten und an eine Datenverarbeitungsvorrichtung weiterleitet.

10. Klimatisierungsvorrichtung nach einem der vorangehenden Ansprüche, **dadurch gekennzeichnet, dass** eine Detektionseinheit (48) vorgesehen ist, die den Probenzustand bewertet und das Analyseergebnis auf einer Datenverarbeitungseinheit ablegt.

## Claims

1. Air-conditioning space (10) for storing samples in a time-controlled manner, comprising an automatic supply device (22) for supplying sample containers (18) to a climatically enclosed space (12) having at least one wall (43), wherein a sample container (18) can be introduced through an opening (40, 42) into the climatically enclosed space (12), wherein the supply device (22) has at least one drive and control unit and an incubator receptacle (30) for receiving the samples (18) is provided within the climatically enclosed space (12), wherein the supply device includes an automatic supply arm (22, 24) which grips a sample container (18) from a pick-up position outside the climatically enclosed space (12) and places the sample container (18) there at a unique placement position in an incubator receptacle (30), which supply arm (22, 24) is arranged outside the air-conditioning space (12), and the sample containers (18) can be removed individually from the climatically enclosed space (12) by means of the supply arm (22, 24), **characterized in that** the opening is formed by a through-channel (40) which at least vertically adjustable.

2. Air-conditioning space according to claim 1, **characterized in that** the supply arm (22, 24) is mounted so as to enable a sample container (18) to be turned during transport of the latter from a pick-up position to a placement position thereof.

3. Air-conditioning space according to one of the preceding claims, **characterized in that** the supply arm (22, 24) is mounted on travel means (28) so as to enable the arm (22, 24) to be moved in the vertical direction and thus to access a placement position on various levels of the incubator receptacle (30).

4. Air-conditioning space according to one of the preceding claims, **characterized in that** a recess (42) is provided in the vertical extension of the side wall (43).

5. Air-conditioning space according to one of the preceding claims, **characterized in that** the incubator receptacle is in the form of a rotatable carousel (30).

6. Air-conditioning space according to claim 5, **characterized in that** a motor is provided outside the enclosed space (12), which motor drives the rotatable carousel (30) via transmission means.

7. Air-conditioning space according to one of the preceding claims, **characterized in that** the climatically enclosed space (12) is a cabinet incubator, a climatic cabinet or an incubator, in particular for cooling and/or heating purposes.

8. Air-conditioning space according to one of the preceding claims, **characterized in that** the pick-up position is located at a connection point (20) to a conveyor line (16).

9. Air-conditioning space according to one of the preceding claims, **characterized in that** a bar code reader (36) is provided which is adapted to evaluate information stored in the bar code and to transmit such information to a data processing device.

10. Air-conditioning space according to one of the preceding claims, **characterized in that** a detection unit (48) is provided which assesses the state of a sample and stores the analysis result in a data processing unit.

## Revendications

1. Chambre de climatisation (10) pour le stockage d'échantillons avec contrôle du temps, comprenant un dispositif d'amenée (22) automatique de récipients à échantillons (18) dans une chambre climatique fermée (12), laquelle présente au moins une paroi (43) et dans laquelle un récipient à échantillons (18) peut être introduit par une ouverture (40, 42) dans la chambre climatique fermée (12), dans laquelle le dispositif d'amenée (22) comprend au moins une unité d'entraînement et de commande, et un système de réception d'incubateur (30) est prévu à l'intérieur de la chambre climatique fermée (12) pour la réception des échantillons (18), dans laquelle le dispositif d'amenée présente un bras d'amenée (22,24) automatique qui saisit un récipient à échantillons (18) se trouvant dans une position de réception à l'extérieur de la chambre climatique fermée (12), et dépose le récipient à échantillons (18) dans un système de réception d'incubateur (30) dans une position de dépôt définie, dans laquelle le bras d'amenée (22, 24) est agencé à l'extérieur de la chambre de climatisation (12), et les récipients à échantillons (18) peuvent être prélevés individuellement de la chambre climatiquement fermée (12) au moyen du bras d'amenée (22, 24), **caractérisée en ce que** l'ouverture est formée par un canal de passage (40) qui est mobile au moins verticalement.

2. Chambre de climatisation selon la revendication 1, **caractérisée en ce que** le bras d'amenée (22, 24) est monté de telle manière qu'un récipient à échantillons (18) est amené à tourner de la position de réception à la position de dépôt pendant le transport.

3. Chambre de climatisation selon l'une quelconque des revendications précédentes, **caractérisée en ce que** le bras d'amenée (22, 24) est agencé sur un moyen de déplacement (28), ce qui a pour effet que ledit bras est mobile dans la direction verticale et peut donc accéder à une position de dépôt dans différents plans du système de réception d'incubateur (30).

4. Chambre de climatisation selon l'une quelconque des revendications précédentes, **caractérisée en ce qu'**un évidement (42) est ménagé dans l'étendue verticale dans la paroi (43).

5. Chambre de climatisation selon l'une quelconque des revendications précédentes, **caractérisée en ce que** le système de réception d'incubateur est réalisé sous la forme d'un carrousel rotatif (30).

6. Chambre de climatisation selon la revendication 5, **caractérisée en ce qu'**un moteur est prévu à l'extérieur de la chambre (12) et entraîne le carrousel rotatif (30) par l'intermédiaire de moyens de transmission.

7. Chambre de climatisation selon l'une quelconque des revendications précédentes, **caractérisée en ce que** la chambre climatique fermée (12) est une étuve à incubation, une armoire climatique ou un incubateur, en particulier pour un refroidissement et/ou un chauffage.

8. Chambre de climatisation selon l'une quelconque des revendications précédentes, **caractérisée en ce que** la position de réception se situe dans un point de raccordement (20) sur une ligne de transport (16).

9. Chambre de climatisation selon l'une quelconque des revendications précédentes, **caractérisée en ce qu'**un lecteur de code à barres (36) est prévu, qui analyse les informations mises en mémoire dans le code à barres sur le récipient à échantillons (18) et les transmet à un dispositif de traitement de données.

10. Chambre de climatisation selon l'une quelconque des revendications précédentes, **caractérisée en ce qu'**une unité de détection (48) est prévue, qui évalue l'état de l'échantillon et met en mémoire le résultat de l'analyse sur une unité de traitement de données.
